# EUROPEAN PATENT APPLICATION

(11) **EP 4 686 764 A1**
(43) Date of publication of application: **04.02.2026**
(21) Application number: 24382850.6
(22) Date of filing: 31.07.2024
(51) Int. Cl.: C12Q 1/6883

(54) **IN VITRO METHOD FOR THE DIAGNOSIS AND/OR PROGNOSIS OF OBESITY**

(71) Applicant: Fundación Pública Galega Instituto de Investigación Sanitaria de Santiago de Compostela (FIDIS), 15706 Santiago de Compostela A Coruña (ES); Servizo Galego de Saúde (SERGAS), 15703 Santiago de Compostela, A Coruña (ES); Universidade de Santiago de Compostela (USC), 15872 Santiago de Compostela A Coruña (ES)
(72) Inventor: Al-Massadi Iglesias, Omar, 15706 Santiago de Compostela (ES); Quiñones Téllez, Mar, 15706 Santiago de Compostela (ES); Seoane Camino, Luisa María, 15706 Santiago de Compostela (ES); Prida García, Eva, 15706 Santiago de Compostela (ES)
(74) Representative: Hoffmann Eitle

(57) **Abstract**

The present invention refers to an *in vitro* method for the diagnosis and/or prognosis of obesity.

## Description

### FIELD OF THE INVENTION

The present invention refers to the medical field. Particularly, the present invention refers to an *in vitro* method for the diagnosis and/or prognosis of obesity.

### STATE OF THE ART

The incidence of worldwide obesity has been increasing at an alarming rate in the last few decades in both adults and in children. Childhood obesity is a determining risk factor for the development of obesity in adulthood. Thus, if obesity levels continue to increase, especially at younger ages, their effect on health and longevity in the coming decades could be very adverse.

In fact, obesity has been shown to have a substantial effect on longevity, the average lifespan of severely obese individuals being reduced by 5 to 20 years.

Because early life determinants are a major factor in the rapid increase in obesity, the identification of new therapies that may aid in the prevention or amelioration of food intake disorders in children and adolescents is of vital importance.

So, there is an unmet medical need of finding reliable tools aimed at diagnosing and/or prognosing of obesity, mainly because the levels of childhood obesity are a determining risk factor for the development of obesity in adulthood. The identification of new biomarkers that can help in the diagnosis and prevention of metabolic disorders is therefore of vital importance. The present invention is focused on solving this problem and an innovative approach is herein presented.

### DESCRIPTION OF THE INVENTION

### Brief description of the invention

The present invention refers to an *in vitro* method for the diagnosis and/or prognosis of obesity.

Particularly, the inventors of the present invention evaluated the metabolic role of the *PTK2B* gene in obese children and adolescents by measuring its mRNA expression levels in peripheral blood mononuclear cells and correlating them with different anthropometric and biochemical parameters.

A cross-sectional study was performed involving 94 Caucasian children and adolescents. They were divided into two groups according to BMI. Differences between the groups were assessed mainly using the Student's T test and the Wilcoxon test. To obtain the final results, a multivariate logistic regression was performed.

The results (see the Examples below) showed increased *PTK2B* mRNA expression in children and adolescents with obesity, suggesting that *PTK2B* is involved in the regulation of obesity. Interestingly, a positive correlation has been found between the levels of *PTK2B* with BMI, fat mass, waist circumference, and hormonal parameters such as leptin. In addition, through use of the multivariate logistic model, high levels of *PTK2B* gene expression were determined to be a predictor of obesity development.

So, the first embodiment of the present invention refers to an *in vitro* method for the diagnosis and/or prognosis of obesity, the method comprising assessing the level of expression of the gene *PTK2B* in a biological sample obtained from the subject.

The second embodiment of the present invention refers to the *in vitro* use of the gene *PTK2B,* or of a kit comprising reagents for assessing the level of expression of the gene *PTK2B,* for the diagnosis and/or prognosis of obesity.

In a preferred embodiment, the biological sample is blood, plasma or serum.

In a preferred embodiment, the method comprises: a) Assessing the level of expression of the gene *PTK2B* in a biological sample obtained from the subject and b) wherein if a higher level of expression is identified with respect to a pre-established reference value measured in control subjects who are not suffering from obesity, it is indicative that the subject is suffering or may suffer from obesity.

In a preferred embodiment, the subjects are children and/or adolescents.

The fourth embodiment of the present invention refers to a kit, suitable for the diagnosis and/or prognosis of obesity, which comprises reagents for assessing the level of expression of the gene *PTK2B.*

The present invention also refers to:
A method for detecting biomarkers in a test sample from a human subject suffering from obesity, the method comprising: a) Contacting the test sample with a primer specific to at least the gene *PTK2B,* b) amplifying to produce an amplification product in the test sample; and c) measuring the expression level by determining the level of the amplification product in the test sample.

In a preferred embodiment, the present invention is a computer-implemented invention, wherein a processing unit (hardware) and a software are configured to:
a) Receive expression level values of the gene *PTK2B,*
b) Process the expression level values received for finding substantial variations or deviations, and
c) Provide an output through a terminal display of the variation or deviation of the expression level, wherein the variation or deviation of the expression level indicates whether the patient has or may have obesity.

Finally, the present invention also refers to a computer program or a computer-readable media containing means for carrying out a method defined above.

For the purpose of the present invention the following terms are defined:
- The expression "pre-established reference value", when referring to the level of the biomarkers described in the present invention, refers to the normalized expression of the gene *PTK2B.* A "reference" value can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data.

- By the term "comprising" is meant the inclusion, without limitation, of whatever follows the word "comprising". Thus, use of the term "comprising" indicates that the listed elements are required or mandatory, but that other elements are optional and may or may not be present.
- By "consisting of" is meant the inclusion, with limitation to whatever follows the phrase "consisting of'. Thus, the phrase "consisting of" indicates that the listed elements are required or mandatory, and that no other elements may be present.

### Brief description of the figures

**Figure 1****.** *PTK2B* expression in children with obesity. *PTK2B* mRNA levels in children with normal weight and children with obesity of both sexes. *PTK2B* mRNA levels in girls with normal weight and girls with obesity. *PTK2B* mRNA levels in boys with normal weight and boys with obesity. Data are expressed as mean ± SEM. * p-value< 0.05, *** p-value < 0.001.

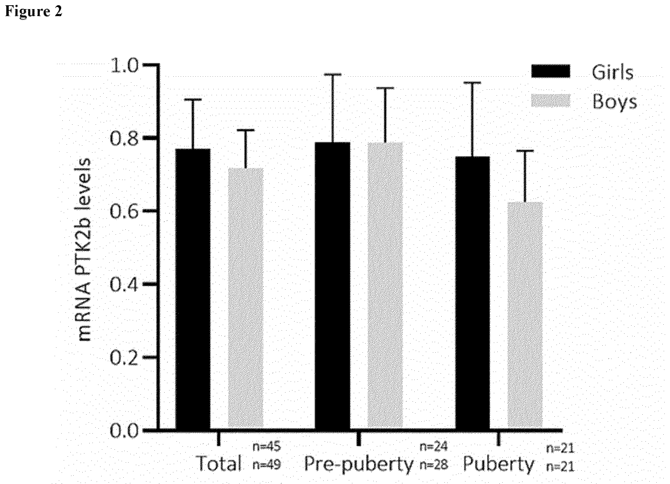

**Figure 2****.** *PTK2B* expression in the study population based on the pubertal stage. *PTK2B* mRNA levels in prepubescent and pubescent individuals of both sexes. *PTK2B* mRNA levels in prepubescent and pubescent girls. *PTK2B* mRNA levels in prepubescent and pubescent boys. Data are expressed as mean ± SEM.
**Figure 3****.** *PTK2B* expression in the study population according to gender. PTK2B mRNA levels in prepubescent girls and boys. *PTK2B* mRNA levels in pubescent girls and boys Data are expressed as mean ± SEM.
**Figure 4****.** Correlation analysis of the study population. Bivariate correlation between *PTK2B* gene expression and BMI **(A),** Waist circumference **(B),** Fat mass **(C)** and Leptin **(D).** BMI: Body mass index.
**Figure 5****.** Odds Ratio Plot of adjusted binary logistic regression model. HDL: high density lipoprotein; HOMA: homeostatic model assessment; TSH: thyroid stimulating hormone.
**Figure 6****.** Predictions plot of obesity by changes in the expression of *PTK2B.*

### Detailed description of the invention

The present invention is specifically illustrated by means of the following Examples without the intention of limiting the scope of protection to what it is described therein.

### Example 1. Materials and Methods

### Example 1.1. Cross-Sectional Study

A cohort of patients was selected including a total of 94 Caucasian children and adolescents (3 - 15 years). Enrolment onto the study occurred between May 2006 and February 2016. The patients were recruited by the Investigation in Nutrition, Growth and Human Development Unit of Galicia (Clinical University Hospital of Santiago de Compostela). This cohort was classified according to body mass index (BMI) as children with obesity (n = 49) or normal weight (n = 45) using age and sex-specific BMI cut-off points of the International Obesity Task Force, equivalent to adult values of 25 kg/m2 for overweight and 30 kg/m2 for obesity 30.

### Example 1.2. Ethical considerations

This is a cross-sectional study, and it adhered to the principles outlined in the Declaration of Helsinki and all procedures were approved by the Central Ethics and Research Committee of the Galician Autonomous Community (code 2013/256). The inclusion of all the children in the study was obtained with written, informed and signed parental consent. Individuals who had taken any medication or had any chronic disorder that could potentially influence the study outcomes were excluded from participation in the study.

### Example 1.3. Clinical Examination and Blood Sampling

Anthropometric measurements were taken in the morning by a single paediatrician with participants wearing only underwear and no shoes. Body weight was recorded using a digital electronic balance (Seca mod. 813. gmbh & CO) with an accuracy of 0.1 kg, while height was measured with a calibrated wall-mounted stadiometer (Seca mod. 213. gmbh & CO) with an accuracy of 0.1 cm. BMI was calculated by dividing the weight in kilograms by the square of the height in meters. Waist circumference (WC) was measured in fasting state by applying an inelastic tape horizontally midway between the lowest rib margin and the iliac crest of the standing child at the end of a gentle expiration.

Whole-body measurement is performed to determine bone mass, non-bone fat-free mass, and fat mass. Using the General Electric LunarEncore^{®} DEXA: X-ray source (38keV and 70keV photons).

Clinical examinations were conducted by trained paediatricians using standardized methods. Pubertal development was performed using Tanner's criteria, according to gender i.e., stage 1 is prepubertal and stages 2 to 5 pubertal.

Blood samples were collected after a 12-hour overnight fasting period.

### Example 1.4. Biochemical assays

Plasma glucose, total cholesterol, and triglyceride (TG) levels were assessed using the Advia 2400 Chemistry System from Siemens Healthcare Diagnostics, Erlangen, Germany. The levels of low-density lipoprotein (LDL) cholesterol and high-density lipoprotein (HDL) cholesterol were measured using the SAS-3 Cholesterol Profile kit from Helena Biosciences Europe, Gateshead, UK. Plasma leptin was determined using a commercial ELISA kit from DRG International, Springfield Township, NJ, USA.

Furthermore, plasma levels of insulin, thyroid-stimulating hormone (TSH), free triiodothyronine (fT3), free thyroxine (fT4), oestradiol, testosterone, follicle-stimulating hormone (FSH), and vitamin-D were determined using a chemiluminescence immunoassay on the Advia Centaur XP analyzer, also from Siemens Healthcare Diagnostics, Erlangen, Germany. Plasma insulin-like growth factor 1 (IGF-1) was analyzed using a chemiluminescence immunoassay on the Immulite 2000 analyzer, also from Siemens Healthcare Diagnostics, Erlangen, Germany. Lastly, circulating free fatty acids (FFA) were measured using a colorimetric kit following the instructions from Wako Chemicals GmbH, Neuss, Germany.

### Example 1.5. PBMCs isolation from peripheral human blood

PBMCs were isolated from the serum-free blood. The blood was diluted 1:2 with PBS without calcium and magnesium. Five millilitres (mL) of the solution were carefully layered avoiding any mixing in 4 mL of PolymorphPrep solution (Progen) and centrifuged for 30 min at 500 x g for gradient separation. Both cell-containing rings indicative of PBMCs were transferred into a new tube, adding PBS and it was centrifuged for 10 min at 500 x g. The cell pellet was resuspended again with centrifuging for 10 min at 500 x g. This was followed by another fill-up of PBS and centrifuging for 10 min at 500 x g. The cell pellet was resuspended with 750 µL of TRIzol reagent.

### Example 1.6. SYBR Green One-step RT - qPCR

The extraction of mRNA was carried out by resuspending the sample in TRIzol to which chloroform (VWR chemicals) was added, vortexed and centrifuged at 12000 x g for 15 minutes at 4°C. The aqueous phase was deposited, and the same amount of 70% ethanol (VRW chemicals) was added, both phases being mixed and transferred to a column (RNA extraction kit, Omega Bio Tek). Finally, 40 uL of H20 pure free RNAse was added and centrifuged at 14000 x g for 2 minutes at 21°C.One-step RT - qPCR was carried out in 10 µL reaction volumes containing 10 ng of RNA template, (the RNA concentration was determined spectrophotometrically with Nanodrop (Thermo Scientific NanoDrop), 0.4 µL of 10 µM forward primer, 0.4 µL of 10 µM of reverse primer, 0.4 µL of NZYRT mix, 5 µL One - step NZYSpeedy qPCR Probe master mix (2x) (NYZtech, Lisbon, Portugal) and 1.8 µL of nuclease-free water. In addition, a calibration curve with varying concentrations was used as an amplification control.

After an initial reverse transcription period of 15 min at 50°C, and a polymerase activation of 2,5 minutes at 95°C, the following steps were repeated for 40 cycles in a 96 wells thermal cycler (QuantStudio 3, applied biosystems, Thermo Fisher Scientific): 5 seconds at 95°C and 45 seconds at 60°C followed by a melt curve stage of 15 seconds at 95°C, 1 minute at 60°C and 1 second at 95°C.

The primers employed were designed using exon-boundary-specific gene expression assays using the online tools provided by the PRIMER-blast program (https://www.ncbi.nlm.nih.gov/tools/primer-blast/). The control housekeeping gene used was β-actin. The list of utilized primers is provided in **Table 1.**

**Table 1**

| ID | Name | Sequence 5'-----3' | Product length |
|---|---|---|---|
| 1 | β - actin Human Forward | CACAGAGCCTCGCCTTTGC | 286 |
| 2 | β - actin Human Reverse | CCACCATCACGCCCTGG | |
| 3 | PTK2b Human Forward | TTAACCAATCCCCGGGAACC | 386 |
| 4 | PTK2b Human Reverse | TTCAAACAAGCCCCGTTCCA | |

### List of primers

### Example 1.7. Statistical analysis

To describe the sample, mean and standard deviation or median and interquartile rank (depending on its adjustment to a normal distribution by Shapiro - Wilk normality test) were calculated for the quantitative variables. Frequencies and percentages were calculated for the qualitative variables. To carry out the comparison between groups, the T-test or Mann-Whitney test and Chi-Squared Test were used. To study the association between PTK2B gene expression and biochemical and anthropometric variables Pearson's chi-squared test (P), Wilcoxon test (W), T-test (T), or Welch Two Sample t-test (We) were used.

**Table 2**

| | **Lean (n=44)** | **Obesity (n=49)** | ***p*** | **TEST** |
|---|---|---|---|---|
| Age (year) | 10.2(3.7) | 11.3(2.9) | 0.2061 | W |
| Sex (girl:boy) | 20:25 | 25:24 | 0.5238 | P |
| Sexual development (prepuberty:puberty) | 25:20 | 23:26 | 0.4038 | P |
| Tanner scale | 1.9 (1.3) | 2.3 (1.5) | 0.5072 | W |
| **Weight (kg)** | **37.3 (14.5)** | **62.0 (19.0)** | **<0.0001** | **W** |
| Height (cm) | 139.3 (22.4) | 149.2 (15.6) | 0.0741 | W |
| **BMI (kg/m²)** | **18.2 (2.8)** | **27.1 (4.3)** | **<0.0001** | **W** |
| **Waist circumference (cm)** | **67. (12.4)** | **90.8 (11.7)** | **<0.0001** | **W** |
| **Fat mass (g)** | **12000 (6459.6)** | **22000 (7936.6)** | **<0.0001** | **W** |
| Muscle mass (g) | 25000 (7930) | 29000 (7854.8) | 0.1363 | W |
| Glucose (mg/dL) | 78.8 (7.2) | 80.1 (7.5) | 0.4117 | T |
| **Insulin (mUI/L)** | **7.9 (4.0)** | **14.3 (7.8)** | **<0.0001** | **W** |
| **HOMA index** | **1.6 (0.8)** | **2.9 (1.8)** | **<0.0001** | **W** |
| IGF-1 (ng/mL) | 306.5 (164.0) | 353.0 (172 0) | 0.6244 | W |
| **Triglycerides (mg/dL)** | **58.1 (34.1)** | **70.0 (38.1)** | **0.0328** | **W** |
| Free fatty acids (mg/dL) | 11.6 (5.7) | 11.4 (4.6) | 0.6880 | W |
| Total cholesterol (mg/dL) | 162.3 (29.6) | 157.5 (34.3) | 0.3363 | W |
| **LDL - cholesterol (mg/dL)** | **98.2 (28.9)** | **90.6 (35.2)** | **0.0314** | **W** |
| **HDL - cholesterol (mg/dL)** | **55.0 (11.9)** | **47.5 (12.5)** | **0.0004** | **W** |
| **Maximum blood pressure** | **105.8 (8.7)** | **114.6 (11.6)** | **0.0004** | **W** |
| **Minimum blood pressure** | **60.1 (6.7)** | **67.3 (10.2)** | **0.0001** | **We** |
| Heart rate | 85.5 (13.6) | 85.7 (16.0) | 0.9728 | W |
| **Leptin (ng)** | **6.9 (5.9)** | **13.5 (11.5)** | **0.0004** | **W** |
| FSH (mUI/L) | 3.1 (2.1) | 3.2 (2.1) | 0.8848 | W |
| TSH (mUI/L) | 2.4 (1.0) | 2.7(1.1) | 0.1568 | W |
| fr3 (ng/dL) | 4.3 (0.4) | 4.2 (0.4) | 0.5445 | W |
| fT4 (ng/dL) | 1.2 (0.1) | 1.2 (0.1) | 0.2442 | W |
| Estradiol (pg/mL) | 31.6 (27.8) | 30.8 (38.3) | 0.5513 | W |
| Testosterone (ng/mL) | 0.8 (1.1) | 0.5 (0.6) | 0.1074 | W |
| Progesterone (ng/mL) | 0.9 (0.6) | 0.9 (1.4) | 0.2497 | W |
| Vitamin - D (ng/mL) | 18.4 (5.3) | 18.0 (6.1) | 0.3896 | W |

| | | | | |
|---|---|---|---|---|
| *Summary of the anthropometric and biochemical characteristics of the participants in the cross-sectional study. Values are presented as mean (SD). Differences between groups were analyzed by Pearson's Chi-squared test (P), Wilcoxon rank sum test (W), t-test (T), or Welch Two Sample t-test (We). Bold values mean significant statistical differences: Weight, Body Mass Index, Waist circumference, Fat mass, Insulin, HOMA index, LDL-cholesterol, HDL- cholesterol, Maximum blood pressure, Minimum blood pressure and Leptin. HDL: high density lipoprotein; HOMA: homeostatic model assessment; LDL: low density lipoprotein.* | | | | |

To study the main objective, a multivariate logistic regression was performed. The model was constructed from univariate regression analysis with each dependent variable. Candidate variables considered were those whose p-value was less than 0.20, or those considered relevant to the researcher's assessment. Subsequently, the final logistic regression model was determined based on the Akaike Information Criterion (AIC).

Statistical packages used to perform the statistical analysis were: GraphPad Prism software (GraphPad Software, 2023) and R software (R Core Team, 2023),
The statistical significance is indicated as follows: *p-value < 0.05, **p-value < 0.01, and ***p-value< 0.001.

### Example 2. Results

### Example 2.1. PTK2B gene expression is upregulated in children and adolescents with obesity

Obesity causes the expected increase in weight, BMI, waist circumference, fat mass, HOMA index, insulin levels, triglycerides (TG), low density lipoprotein (LDL), maximum and minimum blood pressure and leptin compared to control group **(Table 1).** Conversely other circulating factors that are considered beneficial against the disease such as high-density lipoprotein (HDL) are significantly decreased in obese children compared to lean children **(Table 1).**

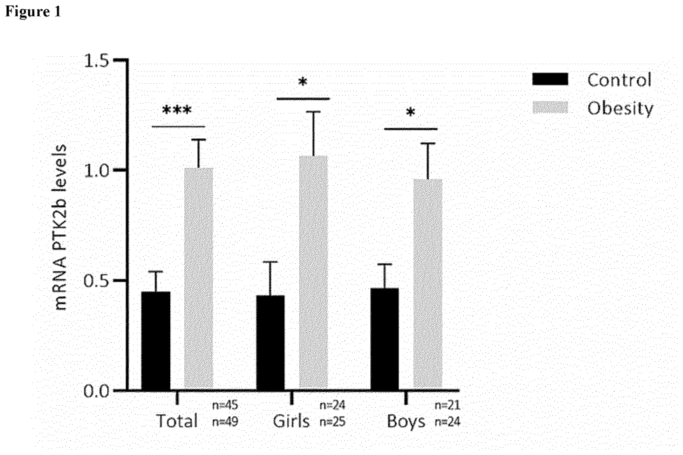

We speculate that the levels of this tyrosine kinase might be increased in human obesity. Considering the difficulty in measuring Pyk2 levels directly from plasma and the wide use of PBMCs as a reliable surrogate marker of tissue expression, we measured *PTK2B* gene levels from PBMCs across our cohort. Our results show that *PTK2B* expression is significantly elevated in obese children compared to the lean controls (**Figure 1A**).

The dataset was further scrutinized, with analyses conducted separately in girls and boys. In both groups, girls and boys presenting obesity, *PTK2B* expression showed a notable increase (**Figure 1A** ). Therefore, this data indicates that the upregulation of PTK2B gene in children with obesity is independent of gender.

To assess the potential impact of the sexual development stage on *PTK2B* expression, we conducted an analysis based on the pubertal stage. Regardless of presenting obesity or normal weight, both children (pre-pubertal stage) and adolescents (pubertal stage) exhibited comparable gene expression patterns (**Figure 1B**)**.**

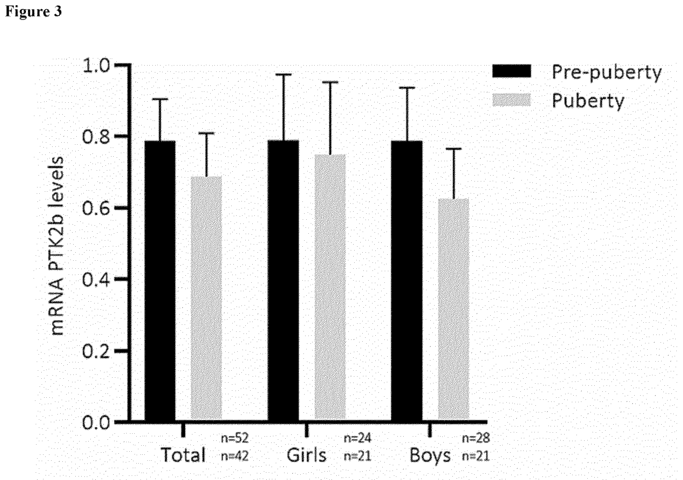

Finally, to explore potential gender-specific variations in our cohort independent of the state of obesity, we compared the levels of PTK2B between girls and boys in the total population (with and without obesity). On completing this comparison, we were unable to find any sexual dimorphism in *PTK2B* expression. In addition, when we divided the population according to both the stage of puberal maturation and by sex, we found that the *PTK2B* gene expression in both sexes is also similar in different stages of sexual development (**Figure 3**).

### Example 2.2. PTK2B gene correlates with adiposity and leptin levels in children and adolescents

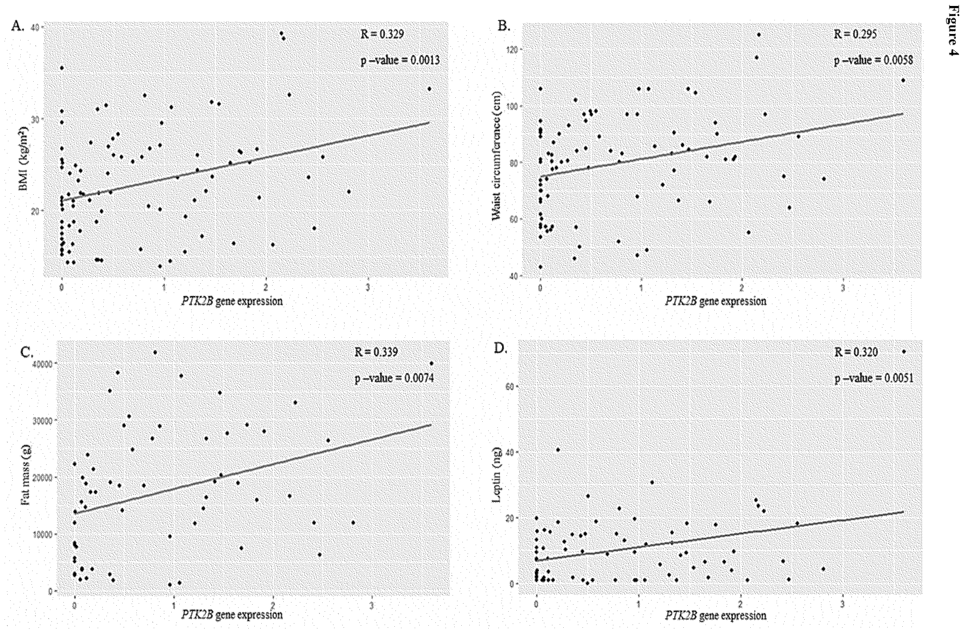

Once we found that *PTK2B* expression is upregulated in obesity, the next step was to correlate these levels with the anthropometric and biochemical factors. The correlation study was performed by avoiding categorical variables. Our results showed a significant positive correlation of *PTK2B* levels and BMI, fat mass, waist circumference and leptin levels (**Table 3** and **Figure 4****).**

**Table 3**

| | **Pearson R²** | **P-value** |
|---|---|---|
| Age (years) | -0.009 | 0.9259 |
| Tanner scale | -0.021 | 0.6541 |
| Weight (kg) | 0.195 | 0.0619 |
| Height (cm) | -0.015 | 0.5738 |
| **BMI (kg/m²)** | **0.329** | **0.0013** |
| **Waist circumference (cm)** | **0.295** | **0.0058** |
| **Fat mass (g)** | **0.339** | **0.0074** |
| Muscle mass (g) | 0.049 | 0.7070 |
| Glucose (mg/dL) | 0.031 | 0.7663 |
| Insulin (mUI/L) | 0.067 | 0.7663 |
| HOMA index | 0.066 | 0.5738 |
| IGF-1 (ng/mL) | -0.087 | 0.4538 |
| Triglycerides (mg/dL) | -0.127 | 0.2340 |
| Free fatty acids (mg/dL) | 0.031 | 0.7824 |
| Total cholesterol (mg/dL) | -0.018 | 0.8665 |
| LDL - cholesterol (mg/dL) | 0.089 | 0.4243 |
| HDL - cholesterol (mg/dL) | -0.018 | 0.8711 |
| Maximum blood pressure | 0.016 | 0.3473 |
| Minimum blood pressure | 0.119 | 0.2880 |
| Heart rate | 0.042 | 0.7077 |
| **Leptin (ng)** | **0.320** | **0.0051** |
| FSH (mUI/L) | 0.020 | 0.8710 |
| TSH (mUI/L) | -0.084 | 0.4404 |
| fT3 (ng/dL) | -0.005 | 0.9662 |
| fT4 (ng/dL) | -0.037 | 0.7667 |
| Estradiol (pg/mL) | -0.104 | 0.3713 |
| Testosterone (ng/mL) | -0.156 | 0.1885 |
| Progesterone (ng/mL) | -0.051 | 0.7621 |
| Vitamin - D (ng/mL) | 0.003 | 0.981 |

| | | |
|---|---|---|
| *Correlations made by Pearson Correlation Coefficient. Bold values mean significant statistical differences with a p-value < 0.05.* | | |

It can be seen that these results fit well with our hypothesis that due to high BMI, waist circumference and high fat mass are the terms that define obesity are positively correlated with PTK2B levels. Similarly, obesity is characterized by a state of leptin resistance when the level of this hormone is high and here, we show that *PTK2B* is positively correlated with this endocrine factor. This is an interesting and significant finding that is well worth highlighting due to it being the first time that *PTK2B* is correlated with these anthropometric factors and with the endocrine factor leptin.

### Example 2.3. Obesity in children is associated with PTK2B gene expression, HDL - cholesterol, testosterone and height

To elucidate which factors or variables can explain the occurrence of obesity among children and adolescents in our study population, a binary logistic regression was conducted, incorporating both biochemical and anthropometric parameters, as well as an assessment of the expression of *PTK2B* gene. We performed a selection of the variables by logistic regression using simple models comparing all variables with the presence of obesity. The selection criteria for this model included a variable when the significance level fell strictly below 0.2 based on simple logistic regression and criteria provided by the research. **(Table 4;** p-value).

**Table 4**

| **Variables** | **OR (CI 95%)** | **P-value** |
|---|---|---|
| Sex (girls:boys) | 0.77 (0.34 - 1.73) | 0.524 |
| **Age (years)** | **1.41 (0.93 - 2.18)** | **0.107** |
| Sexual development (prepuberty: puberty) | 1.41 (0.63 - 3.21) | 0.404 |
| Tanner scale | 1.18 (0.88 - 1.61) | 0.271 |
| **Weight (kg)** | **1.10 (1.06 - 1.15)** | **<0.001** |
| **Height (cm)** | **1.03 (1.01 - 1.05)** | **0.019** |
| **BMI (kg/m²)** | **2.85 (1.90 - 5.34)** | **<0.001** |
| **Waist circumference (cm)** | **1.23 (1.14 - 1.38)** | **<0.001** |
| **Fat mass (g)** | **1.00 (1.00 - 1.00)** | **<0.001** |
| **Muscle mass (g)** | **1.00 (1.00 - 1.00)** | **0.020** |
| Glucose (mg/dL) | 1.02 (0.97 - 1 09) | 0.408 |
| **Insulin (mUI/L)** | **1.20 (1.10 - 1.33)** | **<0.001** |
| **HOMA index** | **2.38 (1.57 - 3.92)** | **<0.001** |
| **IGF-1 (ng/mL)** | **1.00 (1.00 - 1.00)** | **0.184** |
| **PTK2b gene expression** | **2.79 (1.54 - 5.55)** | **0.002** |
| **Triglycerides (mg/dL)** | **1.01 (1.00 - 1.02)** | **0.128** |
| Free fatty acids (mg/dL) | 0.99 (0.91 - 1.07) | 0.840 |
| Total cholesterol (mg/dL) | 1.00 (0.98 - 1.01) | 0.474 |
| LDL - cholesterol (mg/dL) | 0.99 (0.98 - 1.01) | 0.264 |
| **HDL - cholesterol (mg/dL)** | **0.95 (0.91 - 0.98)** | **0.007** |
| **Maximum blood pressure** | **1.09 (1.04 - 1.15)** | **<0.001** |
| **Minimum blood pressure** | **1.11 (1.05 - 1.18)** | **<0.001** |
| Heart rate | 1.00 (0.97 - 1.03) | 0.947 |
| **Leptin (ng)** | **1.13 (1.06 -1.22)** | **0.001** |
| FSH (mUIL) | 1.01 (0.83 - 1.23) | 0.941 |
| **TSH (mUI/L)** | **1.33 (0.91 - 2.00)** | **0.151** |
| fT3 (ng/dL) | 0.58 (0.19 - 1.71) | 0.330 |
| **fT4 (ng/dL)** | **0.07 (0.00 - 2.06)** | **0.134** |
| Estradiol (pg/mL) | 1.00 (0.99 - 1.00) | 0.910 |
| **Testosterone (ng/mL)** | **0.71 (0.40 - 1.13)** | **0.183** |
| Progesterone (ng/mL) | 1.04 (0.68 - 1.70) | 0.850 |
| Vitamin - D (ng/mL) | 0.99 (0.92 - 1.06) | 0.776 |

| | | |
|---|---|---|
| *Selection of variables in obesity. Note. Univariated logistic regressions. OR: the odds ratio. CI 95%: Confidence Interval 95%. P-value. Bold values mean those variables with a significance level lower to 0.2. HDL: high density lipoprotein; HOMA: homeostatic model assessment; TSH: thyroid stimulating hormone.* | | |

Focusing on the results of the univariate logistic regressions, the variables with a p-value higher than 0.2 were eliminated. The variables eliminated were sex, sexual development, Tanner scale, LDL - cholesterol, total cholesterol, free fatty acids, glucose, estradiol, heart rate, fT3, vitamin D, progesterone, and follicle-stimulating hormone (FSH). In addition, the variables highly related to obesity and constitutive of the meaning of the term were eliminated to avoid redundancy, and, in addition, to try to give prominence or statistical space to other factors. Therefore, the variables eliminated were weight, BMI, waist circumference, fat mass, and muscle mass.

Next, we performed an adjusted logistic model as the optimal model that would best explain obesity in children based on the lowest AIC value (**Table 5**). This model includes *PTK2B* gene, HDL, testosterone, HOMA index, height, maximum blood pressure, and thyroid stimulating hormone (TSH) (**Table 5**).

**Table 5**

| | _{c}**OR (CI 95%)** | **_{c}P-value** | **ₐOR (CI 95%)** | **ₐP-value** |
|---|---|---|---|---|
| PTK2b gene | **2.79 (1.54 - 5.55)** | **0.002** | **2.05 (1.27 - 3.32)** | **0.003** |
| HDL - cholesterol | **0.95 (0.91 - 0.98)** | **0.007** | **0.57 (0.35 - 0.94)** | **0.027** |
| Testosterone | **0.71 (0.40 - 1.13)** | **0.183** | **0.56 (0.33 - 0.93)** | **0.026** |
| Height | **1.03 (1.01 - 1.05)** | **0.019** | **1.87 (1.06 - 3.32)** | **0.032** |
| HOMA index | **2.38 (1.57 - 3.92)** | **<0.001** | 1.52 (0.86 - 2.70) | 0.149 |
| Maximum blood pressure | **1.09 (1.04 - 1.15)** | **<0.001** | 1.69 (1.00 - 2.87) | 0.052 |
| TSH | 1.33 (0.91 - 2.00) | 0.151 | 1.43 (0.90 - 2.42) | 0.123 |
| **AIC** | | | 93.15 | |

| | | | | |
|---|---|---|---|---|
| *Univariated and multivariated regression model of obesity status in the selected variables. Note: Bold values mean significant statistical differences. cOR: Crude Odds Ratio, univariated logistic regressions. cP-value: p-value of cOR. aOR: Adjusted Odds Ratio, multivariated logistic regressions. aP-value: p-value of aOR. CI 95%: Confidence Interval 95%.* | | | | |

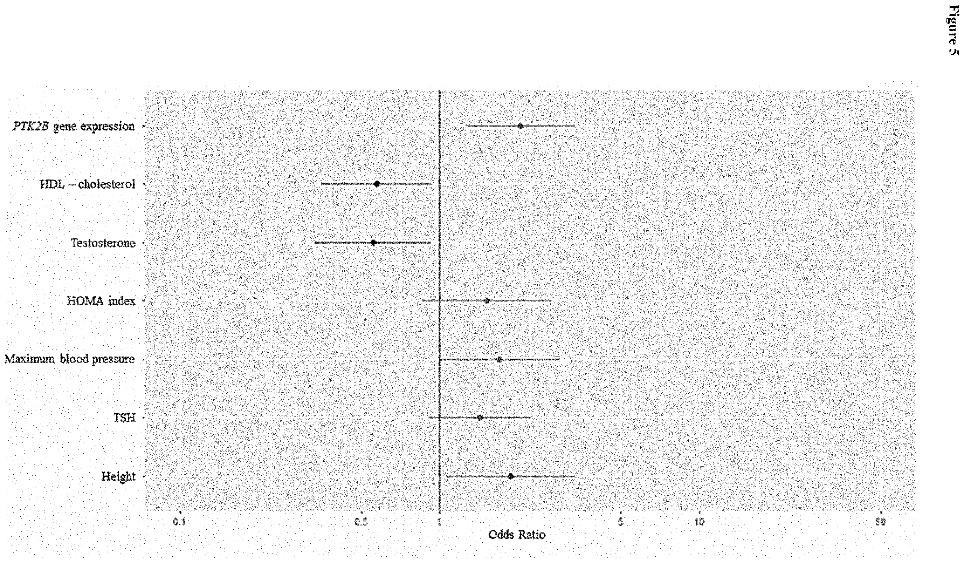

Data were standardized by scaling the values of variables to achieve a mean of "0" and a standard deviation of " 1". Variables with an odds ratio greater than 1 indicate a risk of obesity: conversely, variables with an odds ratio of less than 1 play a protective role against obesity. It is important to note that in this final adjusted model only the statistically significant factors are the ones that can act as predictors of the disease. Therefore, these variables are *PTK2B,* HDL cholesterol, testosterone, and height. For every 1-unit increase in the expression of the *PTK2B* gene, there is an associated 1.80-unit increase in the risk of obesity. Similarly, a 1-unit increase in HDL - cholesterol corresponds to a protective factor of 1.72 units against obesity. In addition, a 1-unit increase in testosterone indicates a protective factor of 1.45 units against obesity. About height, a 1-unit corresponds to a protective factor of 1.79 units against obesity **(Table 5** and **Figure 5****).**

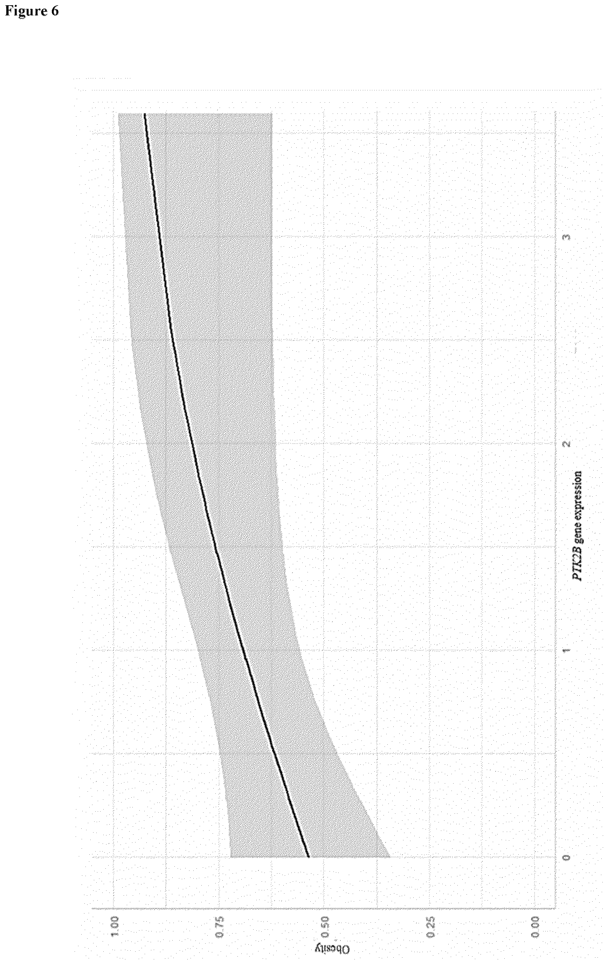

Finally, we compare the **PTK2B** expression with the degree of obesity and with this model's information, we predict that the increased-on mRNA levels of **PTK2B** makes an increased probability of developing obesity **(****Figure 6****).**

In conclusion, we have found the first link between Pyk2 gene expression and the different factors associated with obesity such as BMI, waist circumference and leptin levels in humans. Moreover, the *PTK2B* gene could be used as a biomarker in children and adolescents with obesity and our results highlight, for the first time, the significant role of the *PTK2B* gene in pediatric obesity.

## Claims

1. *In vitro* method for the diagnosis and/or prognosis of obesity, the method comprising assessing the level of expression of the gene *PTK2B* in a biological sample obtained from the subject.

2. *In vitro* use of the gene *PTK2B,* or of a kit comprising reagents for assessing the level of expression of the gene *PTK2B,* for the diagnosis and/or prognosis of obesity.

3. *In vitro* method, or *in vitro* use, according to any of the previous claims, wherein the biological sample is blood, plasma or serum.

4. *In vitro* method, or *in vitro* use, according to any of the previous claims, which comprises: a) Assessing the level of expression of the gene *PTK2B* in a biological sample obtained from the subject and b) wherein if a higher the level of expression is identified with respect to a pre-established reference value measured in control subjects who are not suffering from obesity, it is indicative that the subject is suffering or may suffer from obesity.

5. *In vitro* method, or *in vitro* use, according to any of the previous claims, wherein the subjects are children and/or adolescents.

6. Kit, suitable for the diagnosis and/or prognosis of obesity, which comprises reagents for assessing the level of expression of the gene *PTK2B.*
